# EUROPEAN PATENT APPLICATION

(11) **EP 3 366 300 A1**
(43) Date of publication of application: **29.08.2018**
(21) Application number: 16857480.4
(22) Date of filing: 19.10.2016
(51) Int. Cl.: A61K 35/745, A61K 35/74, A61K 47/36, A61P 37/08

(54) **ANTI-ALLERGIC AGENT FOR INFANTS**

(30) Priority: 19.10.2015 JP 2015205950
(71) Applicant: MEIJI CO., LTD, Chuo-ku Tokyo 104-8306 (JP); Shiga University Of Medical Science, Otsu-shi, Shiga 520-2192 (JP)
(72) Inventor: KOSHIDA Shigeki, Otsu-shi Shiga 520-2192 (JP); TERAHARA Masaki, Odawara-shi Kanagawa 250-0862 (JP)
(74) Representative: TBK
(86) International application number: PCT/JP2016/081002
(87) International publication number: WO 2017/069167

(57) **Abstract**

The present invention provides an antiallergic agent which has no side effects, and which improves allergic diseases of infants, particularly newly born infants. The present invention is related to an antiallergic agent for infants which includes bifidobacteria.

SEQUENCE LISTING

<110>  Meiji  Co.,Ltd

<120>  ANTI-ALLERGIC AGENT FOR NEONATAL INFANTS

<130>  W521372

<150>  JP2015/205950
<151>  2015-10-19

<160>  2

<170>  PatentIn version 3.2

<210>  1
<211>  20
<212>  DNA
<213>  Bifidobacterium bifidum

<400>  1
ccacatgatc gcatgtgatt                                                    20


<210>  2
<211>  19
<212>  DNA
<213>  Bifidobacterium bifidum

<400>  2
ccgaaggctt gctcccaaa                                                     19

## Description

### Technical Field

The present invention relates to an anti-allergic agent and anti-allergic composition for infants.

### Background Art

In modern society, increasing numbers of people suffer from an allergic disease, which is called as one of the modern maladies. The allergic disease has multiple causes, including environmental factors such as dust and mites, and food factors. One getting the allergic disease once, is forced to live in a lifestyle that removes these factors of allergy. A drug therapy that administers drugs is available as a method of alleviation of allergic disease (NPL1).

Localized accumulation and activation of mast cells are remarkable in many allergic diseases. Mast cells express high affinity receptors for IgE. Production of IgE specific to certain allergens induces mast cell accumulation, which causes allergen/IgE-mediated crosslinking and degranulation in the mast cells, and produces free inflammatory mediators.

Similarly, infants with undeveloped immune functions, particularly newborns are more susceptible than adults to sepsis and other infections caused by environmental factors such as dust and mites, and food factors. Antibiotics and other drugs are commonly administered to alleviate such conditions.

### Related Art Document

### Non Patent Literature

NPL1: Wakariyasui Kusuri no Chishiki-Shiite Anshin Kusuri no Tsukaikata-, Editor/Author, Ei Ninomiya, Shinnippon-Hoki Publishing, published March 22, 1995, pp. 379 to 395

### Summary of Invention

### Problems that the Invention is to Solve

Methods that alleviate allergic disease by administration of drugs, as described in the NPL1, are effective at alleviating allergic disease, but are not necessarily effective when side effects of drug administration are considered.

Administration of drugs to newborns is particularly of concern because it affects a subsequent growth. Without drug administration, newborns are forced to live in a food and living environment that removes the factors of allergy.

It is accordingly an object of the present invention to provide an anti-allergic agent and an anti-allergic composition for infants with which allergic disease can be ameliorated without causing side effects.

### Means for Solving the Problems

The present inventors conducted intensive studies, and found that a bacterium belonging to genus *Bifidobacterium* (i.e., bifidobacterium) have an anti-allergic effect for infants, particularly newborns. The present invention was completed on the basis of this finding.

Specifically, the present invention is as follows.
1. An anti-allergic agent for infants, comprising a bifidobacterium.
2. The anti-allergic agent according to the item 1, wherein the bifidobacterium is *Bifidobacterium bifidum.*
3. The anti-allergic agent according to the item 1 or 2, wherein the bifidobacterium is *Bifidobacterium bifidum* OLB6378 strain (accession number: NITE BP-31).
4. The anti-allergic agent according to any one of the items 1 to 3, wherein the bifidobacterium is in the form of a heat-treated bacteria.
5. The anti-allergic agent according to any one of the items 1 to 4, wherein the bifidobacterium is applied in an amount of 10⁸ or more per day continuously for at least 1 month.
6. An anti-allergic composition for infants, comprising the anti-allergic agent of any one of the items 1 to 5, and a dextrin.
7. The anti-allergic composition according to the item 6, which is an anti-allergic food composition.
8. A package comprising the anti-allergic composition according to the item 6 or 7 and a packaging material, wherein the anti-allergic composition is packaged in the packaging material.

### Brief Description of Drawings

Fig. 1 shows diagrams illustrating that there is no difference in gestational age (weeks) and birthweight (g) between subject groups.
Fig. 2 shows diagrams illustrating changes in serum IgE (µg/100 ml) in the subject groups.

### Mode for Carrying Out the Invention

The finding that bifidobacterium have an anti-allergic effect for infants, particularly newborns, enabled the present invention to provide a novel anti-allergic agent or a novel anti-allergic composition for infants containing bifidobacterium and having no side effects.

### Bifidobacterium

The bifidobacterium used for the invention is a bacterium belonging to genus *Bifidobacterium,* and the kind and the origin of the bifidobacterium used for the invention are not limited. Specifically, examples of the bifidobacterium include *Bifidobacterium bifidum, Bifidobacterium longum, Bifidobacterium breve, Bifidobacterium adolescentis, Bifidobacterium infantis, Bifidobacterium pseudolongum,* and *Bifidobacterium thermophilum.*

Specific examples of *Bifidobacterium bifidum* include *Bifidobacterium bifidum* OLB6378 strain (accession number: NITE BP-31). The present invention has enabled providing an anti-allergic agent for infants with the use of this bacterial strain.

The present applicant has deposited this bacterial strain at The National Institute of Technology and Evaluation, Patent Microorganisms Depositary. The following are details of the deposit.

The present applicant inventors deposited the Bifidobacterium bifidum OLB6378 strain (Bifidobacterium bifidum OLB6378) under the following conditions.
(1) Name of depositary authority:
   National Institute of Technology and Evaluation, NITE Patent Microorganisms Depositary
(2) Contact:
   2-5-8 Kazusakamatari, Kisarazu-shi, Chiba 292-0818, Japan (Present Address: 122, 2-5-8 Kazusakamatari, Kisarazu-shi, Chiba 292-0818, Japan)
   Phone number: 0438-20-5580
(3) Accession number:
   NITE BP-31
(4) Identification indication:
   *Bifidobacterium bifidum* OLB6378
(5) Date of original deposit:
   October 26, 2004
(6) Date of transfer to an international deposit under the Budapest Treaty:
   January 18, 2006

*Bifidobacterium bifidum* OLB6378 strain is a gram-positive, obligately anaerobic, rod-shaped bacterium isolated from human infant feces. When this bacterium is cultured on a BL agar (Eiken Chemical Co., Ltd.) plate at 37°C for 48 hours under anaerobic conditions using AnaeroPack-Kenki (manufactured by Mitsubishi Gas Chemical Company, Inc.), opaque, circular, hemisphere, glossy colonies are formed.

Also, a PCR product is obtained by PCR using *Bifidobacterium bifidum-specific* primers (Proceedings of 8th Symposium on Intestinal Flora, Molecular Ecological Detection and Identification of Intestinal Microflora, edited by Tomotari Mitsuoka and Takahiro Matsuki), concretely, BiBIF-1: CCA CAT GAT CGC ATG TGA TT (SEQ ID NO:1) and BiBIF-2: CCG AAG GCT TGC TCC CAA A (SEQ ID NO:2), which are species-specific primers in the 16S rRNA region. The strain also has the ability to ferment galactose, glucose, fructose, lactose and gentiobiose.

A culture medium which is generally used as a culture medium for *Bifidobacterium* can be used for culturing the strain of the invention. That is, the culture medium which can be used for the invention is not particularly limited, and any culture medium can be used as long as the culture medium contains a main carbon source as well as a nitrogen source, inorganic substances and other nutrients in predetermined amounts. As the carbon source, lactose, glucose, sucrose, fructose, starch hydrolysates, molasses and the like can be used depending on the assimilation of the strain used. As the nitrogen source, organic nitrogen-containing compounds such as casein hydrolysates, whey protein hydrolysates and soy protein hydrolysates can be used. In addition, meat extract, fish extract, yeast extract or the like is used as a growth stimulator.

The cultivation is preferably conducted under anaerobic conditions, and a known method such as a method in which the strain is cultured while blowing carbon gas can be used. The strain can be cultured also using another method, for example under microaerophilic conditions using a generally used liquid static culture process or the like or under batch culture conditions. The culture temperature is 25 to 50°C, particularly preferably 35 to 42°C. However, the culture temperature of the invention is not limited to the temperatures, and another temperature condition may also be used as long as the strain can grow at the temperature. The pH of the culture medium is preferably kept at 6.0 to 7.0 during the cultivation, but another pH condition may also be used as long as the strain can grow at the pH. The culture period is preferably 3 to 48 hours, further preferably 8 to 24 hours, particularly preferably 10 to 20 hours, but another culture period may also be employed as long as the strain can grow in the culture period.

The bacteria obtained may be contained as a treated bifidobacterium product in an anti-allergic agent or an anti-allergic composition after being processed, as follows. For example, the treated bifidobacterium product may be in the form of a product as cultured, a cultured product after centrifugation or filtration, concentrates of these products, pastes of these products or concentrates, a dried product obtained by using various techniques (for example, a spray dried product, a freeze dried product, a vacuum dried product, and a drum dried product), a liquid product dispersed in a medium, a dilution product obtained after dilution with a diluent, a heat-treated product after a heat treatment (heat-treated bacteria), a photo-irradiated product after exposure to UV light and/or radiation (photo-irradiated bacteria), a chemically treated product after a chemical treatment (with a disinfectant, an antimicrobial agent, or a bacteriostatic agent) (chemically treated bacteria), or a disrupted product obtained after disrupting the dried product with, for example, a mill.

The centrifugation, filtration, concentration, and disruption are performed by using common techniques. The drying may be, for example, vacuum drying, spray drying, freeze drying, or drum drying. The medium, the diluent, the chemical (the disinfectant, the antimicrobial agent, or the bacteriostatic agent) and the like may be known ones, which are appropriately selected and used. In this specification, these products are also collectively called "treated bifidobacterium product", or simply, "treated product."

It was found in the present invention that the bifidobacterium exhibit an anti-allergic effect even after being inactivated by a heat treatment performed, e.g., at 80°C for 10 minutes, as will be described later in Examples. Accordingly, a treated product containing the bacteria of the present invention is useful not only when the bacteria are viable bacteria, but when the bacteria are heat-treated bacteria (for example, bifidobacterium of a form that does not form colonies after a 0.1-ml sample from a heat-treated bifidobacterium suspension (dispersion) is smeared onto a petri dish containing a bifidobacterium growth medium, and cultured under anaerobic conditions).

The bifidobacterium and/or a treated product thereof obtained by using the foregoing methods may be used in the form of viable bacteria or heat-treated bacteria, and may be incorporated in an anti-allergic agent for infants of the present invention either alone or as a mixture of different bifidobacterium or treated bifidobacterium products after being disrupted or without being disrupted.

In the case of viable bacteria, the bacteria can be expected to proliferate in the body (in the intestines) after ingestion. The poor survival of bifidobacterium in the presence of oxygen needs not to be considered with heat-treated bacteria (for example, bifidobacterium of a form that does not form colonies after a 0.1-ml sample from a heat-treated bifidobacterium suspension (dispersion) is smeared onto a petri dish containing a bifidobacterium growth medium, and cultured under anaerobic conditions). This is preferred as it widens the applicable area of the anti-allergic agent for infants of the present invention.

Particularly preferably, the bifidobacterium are heat-treated bacteria killed by a heat treatment. The bifidobacterium undergo changes in cell structure by being heat treated. Presumably, this helps expose the substance that brings about the anti-allergic effect.

The bifidobacterium that proliferated by, for example, being cultured in a medium may be used after removing the medium by using methods such as centrifugation. Here, the anti-allergic effect for infants of the present invention can further improve when the medium components are kept in the bifidobacterium culture without being completely washed away. The bifidobacterium used in the present invention may be, for example, a commercially available bifidobacterium powder product available under the trade name Meiji Bifipure from Meiji Food Materia.

The heat treatment may be performed at a heating temperature of, for example, typically 60 to 300°C, preferably 60°C to 200°C, more preferably 60 to 150°C, further preferably 60 to 140°C, even more preferably 60 to 130°C, yet more preferably 60 to 120°C, further preferably 60 to 110°C, even more preferably 60 to 100°C, yet more preferably 70 to 100°C, further preferably 70 to 90°C, particularly preferably 75 to 85°C.

A heat treatment condition of 60°C or more is preferable because it kills the viable cells of the bifidobacterium. A heat treatment condition of 300°C or less is preferable because it allows the bifidobacterium to remain without being carbonized.

The heat treatment is performed for typically 0.01 to 120 minutes, preferably 0.015 to 60 minutes, more preferably 0.02 to 40 minutes, further preferably 0.025 to 30 minutes, even more preferably 0.03 to 25 minutes, particularly preferably 0.03 to 20 minutes, more particularly a heat treatment of 5 minutes or more. A heat treatment time of 0.1 minutes or more is preferable because it kills the viable cells of the bifidobacterium. A heat treatment time of 120 minutes or less is preferable in terms of inhibiting heat denaturation for efficient killing of viable cells.

In a low-temperature (60 to 100°C) heat treatment, the optimum heat treatment time may be, for example, 0.2 to 120 minutes, preferably 0.2 to 60 minutes, more preferably 0.2 to 40 minutes, further preferably 0.2 to 30 minutes, even more preferably 0.2 to 25 minutes, particularly preferably 0.2 to 20 minutes.

In a high-temperature (100 to 300°C) heat treatment, the optimum heat treatment time may be, for example, 0.01 to 0.5 minutes, preferably 0.015 to 0.5 minutes, more preferably 0.02 to 0.5 minutes, further preferably 0.025 to 0.5 minutes, even more preferably 0.03 to 0.5 minutes, particularly preferably 0.03 to 0.5 minutes.

For example, the heat treatment is preferably performed at 80°C for 10 minutes, or at 90°C for 15 seconds.

The heat treatment method is not particularly limited. For example, the bacteria obtained may be heated under predetermined conditions using a heat sterilizer such as a plate sterilizer, a tubular sterilizer, a direct heating sterilizer, and a jacket-equipped tank.

The amount of bifidobacterium that should be ingested to exhibit the anti-allergic effect for infants of the present invention is, for example, in order of preference, 10⁸ or more/day, 10⁸ to 10¹²/day, 5 × 10⁸ to 5 × 10¹¹/day, 10⁹ to 10¹¹/day, 5 × 10⁹ to 5 × 10¹⁰/day, 6 × 10⁹ to 4 × 10¹⁰/day, or 7 × 10⁹ to 3 × 10¹⁰/day, preferably 8 × 10⁹ to 2 × 10¹⁰/day, further preferably 9 × 10⁹ to 2 × 10¹⁰/day.

The anti-allergic effect for infants can actually be obtained within these ranges of amount of bifidobacterium above. It has been found that the anti-allergic agent for infants of the present invention is a component having a preventive and therapeutic effect, specifically an active ingredient. Accordingly, the anti-allergic agent for infants of the present invention may be used for any purpose, as long as its effect is exhibited.

The anti-allergic agent of the present invention (hereinafter, also referred to simply as "agent of the present invention") has less side effects, and can be continuously ingested by infants, including newborns. The ingestion period of the bifidobacterium of the present invention for exhibiting the anti-allergic effect for infants is, for example, in order of preference, at least 1 month, 1 to 12 months, 1 to 10 months, 1 to 9 months, 1 to 8 months, or 2 to 7 months.

The anti-allergic effect for infants can actually be obtained within these ranges of ingestion period above. Preferably, the bifidobacterium are continuously used for at least 1 month in an amount of at least 10⁸ per day in terms of the number of bacteria, particularly preferably, the bifidobacterium are continuously used for at least 1 month in an amount of at least 10¹⁰ per day in terms of the number of bacteria.

In the present specification, the term "infants" means child of from 0 month age to less than one year age, e.g., child of from 0 month age to 6 months age, and encompasses healthy infants, immature infants, premature infants, and low-birth-weight infants. In the present invention, infants include human infants, unless otherwise specifically stated.

The anti-allergic agent and the anti-allergic composition for infants of the present invention have been shown to exhibit an allergy preventive and therapeutic effect when ingested (administered) by infants, particularly newborns, and when the ingestion (administration) is continued for a time period. Specifically, serum IgE has been shown to significantly decrease as compared to when the anti-allergic agent or the anti-allergic composition for infants of the present invention is not ingested (administered).

In other words, ingestion (administration) of the anti-allergic agent and the anti-allergic composition of the present invention produces an anti-allergic effect in a newborn without causing side effects, and the effect is expected to last for the subsequent growth period. This makes it possible to eliminate and/or reduce the inconvenience incurred to subject, who is forced to live in a food and living environment that removes the factors of allergy.

Newborns have undeveloped immune functions. Ingestion of the anti-allergic agent or the anti-allergic composition of the present invention provides an anti-allergic effect in an early stage of life, particularly in low-birth-weight infants, who are susceptible to infections and allergic disease, and the effect is expected to last for the subsequent growth period. This makes it possible to eliminate and/or reduce the inconvenience incurred to subject, who is forced to live in a food and living environment that removes the factors of allergy.

As used herein, the term "newborns" are of, for example, in order of preference, 0 to 60 days, 0 to 50 days, 0 to 40 days, 0 to 30 days, 0 to 20 days, 0 to 15 days, or 0 to 10 days of age. As used herein, the term "low-birth-weight infants", when applied to humans, are infants with a birthweight of, in order of preference, 300 to 3,000 g, 350 to 2,900 g, 400 to 2,800 g, 450 to 2,700 g, 500 to 2,600 g, or 500 to 2,500 g.

The agent of the present invention may be used by itself, or as an anti-allergic composition for infants of the present invention by being mixed with other components (hereinafter, also referred to as "composition of the present invention"). The agent of the present invention in the composition may have any content as may be decided according to factors such as the intended purpose, use, form, dosage form, symptoms, and body weight. Also, the "composition" may be replaced to "agent".

The content of agent of the present invention in the composition of the present invention may be 0.001 to 90% (w/w), preferably 0.001 to 50% with respect to the total amount of the composition, though the present invention is not limited to these. These contents are preferable for ease of ingestion (administration).

The agent or the composition of the present invention may be administered orally or parenterally (intramuscularly, subcutaneously, intravenously, percutaneously, or as a suppository). Without side effects, as may occur in administration of drugs, administration of the agent or the composition of the present invention is possible. The agent or the composition of the present invention exhibits an anti-allergic effect while promoting other effects, including amelioration of diarrhea, amelioration of constipation, inhibition of proliferation of enteric harmful bacteria, B vitamin production, and digestion and absorption of lactose through decomposition.

Specifically, the agent or the composition of the present invention may be used in the form of a pharmaceutical, or a food or beverage. For example, the agent or the composition of the present invention should exhibit an anti-allergic effect by being directly administered as a pharmaceutical, or by being directly ingested as a food product produced for specific purposes, such as a food for specified health uses, or as a nutritional food product. Examples of such food products produced for specific purposes, and nutritional food products include formula milk, liquid foods, hospital foods, powdered milk for infants, powdered milk for a little child, powdered milk to be used by nursing mothers, supplements, and nutrition enriched food products.

When used as a pharmaceutical, the agent of the present invention may be orally administered in the form of, for example, a tablet, a coated tablet, a capsule formulation, a granule, a powder, a solution, a syrup, and an emulsion preparation. These preparations may be prepared into a composition of the present invention from the main component bacteria and/or treated product of the present invention with a known auxiliary agent commonly used in the field of pharmaceutical preparation, such as dispersants, excipients, binders, disintegrants, lubricants, colorants, flavoring agents, solubilizing agents, suspensions, and coating agents, using an ordinary method, to obtain an oral preparation comprising the composition of the invention.

Preferably, the agent of the present invention is used as composition by being mixed with a dispersant. Examples of the dispersant include, for example, milk proteins such as casein, soy proteins, peptides, amino acids, starches, dextrins, xylans, oligosaccharides, sugars (glucose, lactose, sucrose, galactose, and maltose), and sugar alcohols (trehalose, xylitol, erythritol, palatinose, trehalulose, and xylose). Particularly preferred as the dispersant is a dextrin. Dextrins are preferred dispersants because dextrins allow a powder to granulate, and are easy to handle such as in dispersing or dissolving the agent, in addition to having a long storage capability.

Preferably, the dispersant, particularly the dextrin is granular in shape. By being a granule, a dextrin can have improved solubility, and can be divided in small portions because of easy chargeability. Granular dextrins are also advantageous in terms of production because granular dextrins can be accurately divided by being simply dropped on a packaging material, without producing variation in the mass distribution.

The mass ratio of the agent of the present invention and the dispersant in the composition of the present invention is preferably 1:100 to 1:2, more preferably 1:100 to 1:10, further preferably 1:100 to 1:20. These ranges of mass ratio of the agent of the present invention and the dispersant in the composition of the present invention are preferable because they allow the anti-allergic agent of the present invention to be efficiently dispersed.

For example, for oral administration of the composition of the present invention containing the agent of the present invention and a dextrin, the composition of the present invention may be administered after being packaged into packages with a packaging material in small predetermined portions. In the present invention, it is preferable that the composition of the present invention be packaged into a single package for each dose, or multiple packages for each dose. Particularly preferably, a single package is prepared for each dose.

When adding the agent or the composition of the present invention to a food composition having no side effects, the agent or the composition of the present invention may be ingested in the form of various food and drink products (such as milk, soft drinks, fermented milk, yogurt, cheese, bread, biscuits, crackers, pizza crusts, formula milk, liquid foods, hospital foods, powdered milk for infants, powdered milk for a little child, powdered milk to be used by nursing mothers, and nutrition enriched food products) by being added to these products. The agent and the composition of the present invention may be used directly, or in the form of a common food composition prepared according to an ordinary method, for example, by being mixed with food products or food components. The agent and the composition of the present invention may be in a state of common food and beverages, for example, such as a solid (including a powder, and a granule), a paste, a liquid, and a suspension. In these forms, the agent of the present invention can be ingested without feeling uncomfortable.

The agent or the composition of the present invention also may be used as a composition prepared as a mixture with materials having no side effects, for example, such as water, proteins, carbohydrates, lipids, vitamins, minerals, organic acids, organic bases, fruit juice, and flavors.

Examples of the proteins include animal and plant proteins such as whole powdered milk, powdered skim milk, partially skimmed powdered milk, casein, a whey powder, whey proteins, whey protein concentrates, separated whey proteins, α-casein, β-casein, κ-casein, β-lactoglobulin, α-lactoalbumin, lactoferrin, soy proteins, chicken egg proteins, meat proteins, and hydrolysates thereof; and various components derived from milk, such as butter, lactic minerals, creams, whey, non-protein nitrogen, sialic acid, phospholipids, and lactose. All drugs, and all food and drink products that have been used with no known side effects are applicable. These components may be used in a combination of two or more.

Examples of the carbohydrates include sugars, processed starches (dextrins, soluble starches, British starch, oxidized starches, starch esters, and starch ethers), and dietary fibers.

Examples of the lipids include animal oils, such as lard, fish oil, and fractionated oils, hydrogenated oils, and ester exchange oils thereof; and vegetable oils, such as palm oil, safflower oil, corn oil, canola oil, coconut oil, and fractionated oils, hydrogenated oils, and ester exchange oils thereof.

Examples of the vitamins include vitamin A, carotenes, B vitamins, vitamin C, D vitamins, vitamin E, K vitamins, vitamin P, vitamin Q, niacin, nicotinic acid, pantothenic acid, biotin, inositol, choline, and folic acid.

Examples of the minerals include calcium, potassium, magnesium, sodium, copper, iron, manganese, zinc, and selenium.

Examples of the organic acids include malic acid, citric acid, lactic acid, and tartaric acid. All drugs, and all food and drink products that have been used with no known side effects are applicable.

These components may be used in a combination of two or more.

When the agent or the composition of the present invention is provided as a food product or a medicament, these may be produced by using methods known to a skilled artisan. A skilled artisan would be able to produce a desired food product or a desired medicament by appropriately combining different processes, including mixing the bifidobacterium or the treated product of the present invention with other components, molding, sterilization, fermentation, baking, drying, cooling, granulation, and packaging.

The agent or the composition of the present invention is also applicable to foods with health claims, and hospital foods. The designation "Foods with Health Claims" is a system established to provide coherence to the conventional system of food for specified health uses, in keeping with the recent domestic and foreign trends. The new system is intended to cover not only common food products but other food product forms such as tablets and capsules, and includes two categories: Food for Specified Health Uses (approved case-by-case), and Food with Nutrient Function Claims (standardized). The agent or the composition of the present invention should provide an anti-allergic effect when directly ingested as a food product for specified uses, such as a food for specified health uses, or as a food with nutrient function claims by being contained in these products.

The agent and the composition of the present invention may be added to formula milk to prepare an oral composition for preventing or treating an allergy in infants. The formula milk may be in the form of, for example, formula milk for infants, peptide milk, follow-up milk, growing-up milk, formula milk for low-birth-weight infants, lactose-free powdered milk, low-sodium specialty powdered milk, and a supplement powder for breast milk. However, the formula milk is not particularly limited, as long as the effects and efficacy of the present invention can be expected. The agent and the composition of the present invention may be also added to drinks and foods other than formal milk without any limitation. As the drinks and foods, yogurt, confectionery and the likes are exemplified.

The active ingredient bifidobacterium of the present invention may be used as an additive for pharmaceutical compositions, food and beverages that are commonly consumed, and are believed to involve a few side effects, or compositions with a potential anti-allergic effect. The active ingredient bifidobacterium of the present invention may be orally ingested, with or without a tube.

The active ingredient bifidobacterium of the present invention show the foregoing desirable effects and efficacy in humans and other mammals. Accordingly, the present invention also includes feeds and feed additives containing bifidobacterium as an active ingredient, particularly, powdered milk, and additives for powdered milk for raising mammals.

### Examples

The present invention is described below in greater detail, with reference to the results of testing conducted in Examples and Comparative Examples according to the present invention. It is to be noted that the present invention is not limited by the following.

### Example 1

### Preparation of Composition of the Present Invention (Heat Treatment)

### a) Preparation of Lyophilized Powder of Heat-Treated OLB6378 Strain

Three hundred fifty grams of a raw powder of the *Bifidobacterium bifidum* OLB6378 strain (accession number: NITE BP-31; viable bacteria: 3.9 × 10¹¹ cfu/g; trade name: Meiji Bifipure®, Meiji Food Materia) was stirred and completely suspended in 3,500 ml (10%) of feedstock water that had been brought to 45°C. The bacteria were heated while being stirred, and maintained at 80°C for 10 minutes before being cooled. The resulting suspension of heated bacteria was freeze dried to obtain 300 g of a lyophilized powder of the heat-treated OLB6378 strain. The heat-treated OLB6378 strain did not contain viable bifidobacterium on an MRS agar plate medium. As a rough estimate, 1.37 × 10¹⁴ bifidobacteria (3.9 × 10¹¹ (cfu/g) × 350 (g) = 1.37 × 10¹⁴ cfu) are present in 300 g of the lyophilized powder of the OLB6378 strain. Here, the bacteria count of the heat-treated bacteria is given in terms of the number of viable bacteria (cfu).

### b) Preparation of Composition of the Present Invention (Heat Treatment)

One hundred twenty grams of the lyophilized powder of the heat-treated OLB6378 strain was homogenously mixed with 2,880 g of a granular dextrin (Matsutani Chemical Industry Co., Ltd.), and the mixture was divided in 0.5-g portions as the composition of the present invention. In the composition of the invention, as a rough estimate, 9.13 × 10⁹ bifidobacteria (1.37 × 10¹⁴ (cfu) × [120(g)/300(g)] × [0.5(g)/3,000 (g)] = 9.13 × 10⁹ cfu) are present in the composition of the present invention. Here, the bacteria count of the heat-treated bacteria is given in terms of the number of viable bacteria (cfu).

### Example 2

### Preparation of Composition of the Present Invention (Viable Bacteria)

One hundred twenty grams of a raw powder of the OLB6378 strain shown in Example 1 (viable bacteria: 3.9 × 10¹¹ cfu/g) was homogenously mixed with 2,880g of a granular dextrin (Matsutani Chemical Industry Co., Ltd.). The mixture was then divided into 0.5-g portions as the composition of the present invention. In the composition of the invention, 9.13 × 10⁹ (cfu) of the bifidobacterium cells are approximately present.

### Test Example 1

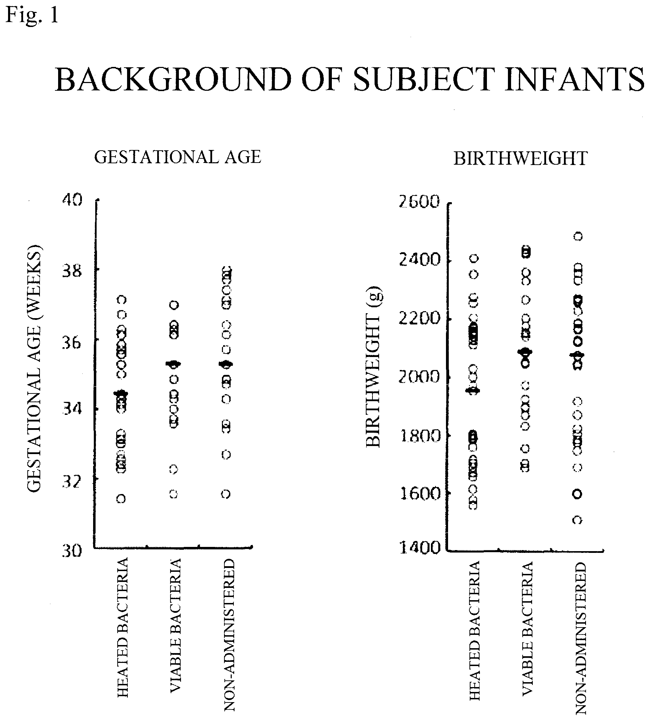

In Test Example 1, groups of subjects were administered with the composition of the present invention (heated bacteria) of Example 1 (composition (heated-bacteria)-administered group), the composition of the present invention (viable bacteria) of Example 2 (composition (viable bacteria)-administered group), and not administered with any composition (control (non-administered) group). The subject groups were chosen from low-birth-weight infants (a birthweight of 2,500 g or less) with the gestational age of 30 to 38 weeks, and divided into an composition (heated bacteria)-administered group (24 subjects), an composition (viable bacteria)-administered group (29 subjects), and a control (non-administered) group (29 subjects). The composition (heated bacteria) in the b) of Example 1 and the composition (viable bacteria) of Example 2 was administered to the composition (heated-bacteria)-administered group and the composition (viable bacteria)-administered group, respectively, twice daily. Each of the composition (heated-bacteria)-administered group and the composition (viable bacteria)-administered group was subjected to the first administration within 48 hours after birth, and the period of administration was 6 months. There was no difference in the gestational age or the birthweight of the subjects between these groups, as shown in Fig. 1.

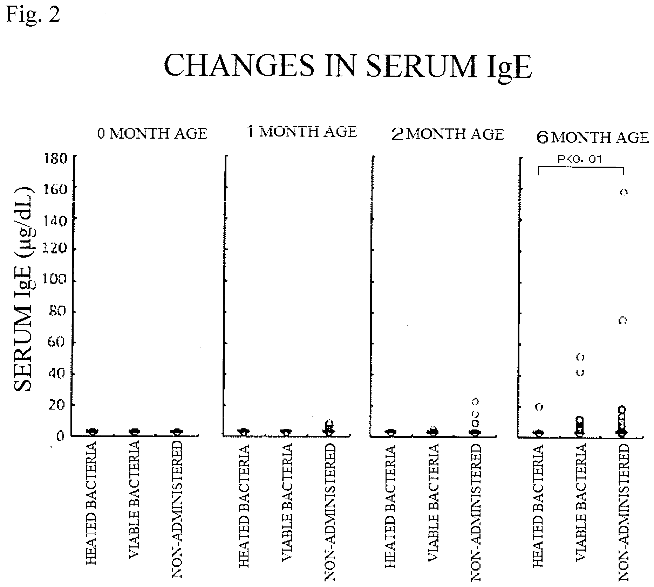

A serum sample was collected from the subjects in each group at 0, 1, 2, and 6 month age, and serum IgE was measured. The measurement of serum IgE was carried out by the nephelometry method (BM6070 by JOEL Ltd.) The results are presented in Fig. 2.

As shown in Fig. 2, the serum IgE at 6 month age was significantly lower in the composition (heated bacteria)-administered group than in the control group. The result demonstrated that the composition of the present invention (particularly, the composition (heated bacteria) of the present invention) was effective at producing an anti-allergic effect in infants, particularly low-birth-weight newborns.

Although the invention has been explained in detail using specific embodiments, it is obvious to one skilled in the art that various changes and modifications can be made without departing from the purpose and the scope of the invention. The present application is based on a Japanese patent application filed on October 19, 2015 (patent application No. 2015-205950), and the entire contents thereof are incorporated in the invention by reference.

### Industrial Applicability

The anti-allergic composition and the anti-allergic composition for infants of the present invention should be useful for preventing or treating allergic diseases without causing side effects. The anti-allergic composition and the anti-allergic composition for infants of the present invention are particularly effective in terms of preventing allergies in newborns, and promoting effective development and growth of infants.

## Claims

1. An anti-allergic agent for infants, comprising a bifidobacterium.

2. The anti-allergic agent according to claim 1, wherein the bifidobacterium is *Bifidobacterium bifidum.*

3. The anti-allergic agent according to claim 1 or 2, wherein the bifidobacterium is *Bifidobacterium bifidum* OLB6378 strain (accession number: NITE BP-31).

4. The anti-allergic agent according to any one of claims 1 to 3, wherein the bifidobacterium is in the form of a heat-treated bacteria.

5. The anti-allergic agent according to any one of claims 1 to 4, wherein the bifidobacterium is applied in an amount of 10⁸ or more per day continuously for at least 1 month.

6. An anti-allergic composition for infants, comprising the anti-allergic agent of any one of claims 1 to 5, and a dextrin.

7. A package comprising the anti-allergic composition according to claim 6 and a packaging material, wherein the anti-allergic composition is packaged in the packaging material.
